# EUROPÄISCHE PATENTSCHRIFT

(11) **EP 0 428 939 B1**
(45) Veröffentlichungstag und Bekanntmachung des Hinweises auf die Patenterteilung: **17.01.1996**
(21) Anmeldenummer: 90121348.8
(22) Anmeldetag: 08.11.1990
(51) Int. Cl.: C07D 311/16

(54) **Cumarinderivate, Verfahren zu ihrer Herstellung, ihre Verwendung und Thiazolyl-essigsäurederivate als Zwischenprodukte**
Cumarin derivatives, process for their preparation, their use and thiazolylacetic acid derivatives as intermediate
Dérivés de coumarine, leur procédé de préparation, leur utilisation et dérivés d'acide thiazolylacétique comme intermédiaires

(30) Priorität: 21.11.1989 DE 3938598; 23.08.1990 DE 4026613
(43) Veröffentlichungstag der Anmeldung: 29.05.1991
(73) Patentinhaber: BAYER AG, D-51368 Leverkusen (DE)
(72) Erfinder: Kuckert, Eberhard, Dr., West Haven, CT 06516 (US); Beck, Gunther, Dr., W-5090 Leverkusen 1 (DE); Seng, Florin, Dr., W-5060 Bergisch Gladbach 2 (DE); Löbberding, Antonius, Dr., W-5600 Wuppertal 1 (DE)

(56) Entgegenhaltungen:
- EP-A- 0 025 136
- EP-A- 0 032 670
- EP-A- 0 056 577
- EP-A- 0 101 897
- DE-A- 2 710 285
- DE-A- 2 712 408
- US-A- 4 547 579
- CHEMICAL ABSTRACTS, vol. 77, no. 6, 7. August 1972, Seite 107, Nr. 36362y, Columbus, Ohio, US; H. UMEMOTO et al.: "Fluorescent whitening agents for synthetic fibers. XIX. Fluorescence of 3-(4'-aminophenyl)-7-aminocoumarins",& NIPPON KAKAKU KAISHI, 1972, (3), 644-9
- Helvetica Chimica Acta, 1970, 53(7), 1963
- Chemicke Zwesti, 1976, 30(2), Seiten 186-187
- Journal für Praktische Chemie, 1982,324(1),Seiten 21- 28

## Beschreibung

Die Erfindung betrifft Cumarinderivate der Formel 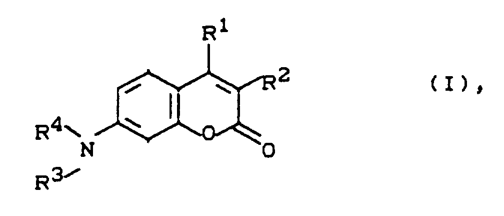 in der
- R¹: Wasserstoff oder Cyano bedeutet,
- R: für Phenyl oder für in 2-, 4- oder 5-Stellung gebundenes Thiazolyl steht,
wobei Phenyl durch Cyano, Amino, -NH-C₁-C₄-Alkyl, -C₁-C₄-Alkyl-NH₂, -C₁-C₄-Alkyl-NH-C₁-C₄-Alkyl, Carboxyl, C₁-C₄-Alkoxy-carbonyl, C₁-C₄-Alkyl- carbonyloxy, Hydroxy, C₁-C₄-Alkylamino-carbonyl oder C₁-C₄-Alkylcarbonyl-amino substituiert ist und zusätzlich durch C₁-C₄-Alkyl, Fluor, Chlor oder Brom substituiert sein kann, und wobei Thiazolyl ein- oder zweifach durch Chlor, Cyano, Carboxyl oder C₁-C₄-Alkoxy-carbonyl substituiert ist, wobei bei zweifacher Substitution die beiden Substituenten verschieden sein können und wobei die 4- und die 5-Stellung gemeinsam einen ankondensierten Benzolring tragen können, der durch Carboxyl, Amino oder Hydroxy substituiert sein kann,
- R³: Wasserstoff, C₁-C₄-Alkyl oder C₁-C₄-Alkoxy-carbonyl-C₁-C₄-alkyl bedeutet und
- R⁴: für C₁-C₄-Alkyl oder Phenylsulfonyl steht, wobei C₁-C₄-Alkyl durch Hydroxy, Amino, Carboxy oder C₁-C₄-Alkoxy-carbonyl substituiert sein kann und Phenyl ein- oder zweifach durch Chlor, Brom oder C₁-C₄-Alkyl substituiert sein kann,
wobei weiterhin R³ und R⁴ gemeinsam mit dem N-Atom, das sie substituieren, einen Morpholinring oder einen Piperazinring, der einen oder zwei Substituenten aus der Gruppe Methyl, Ethyl und Phenyl tragen kann, bedeuten können und
wobei weiterhin einer der Reste R, R³ und R⁴ eine primäre oder sekundäre Aminogruppe, die Hydroxylgruppe, die Carboxylgruppe, Cyano, Acylamino Phenylsulfonylamino oder die C₁-C₄-Alkoxycarbonylgruppe trägt, wobei Cyano oder die C₁-C₄-Alkoxycarbonylgruppe durch Verseifung in die Carboxylgruppe, ebenfalls durch Verseifung die Acylamino- oder Phenylsulfonylaminogruppe in eine Aminogruppe und die Cyanogruppe durch Hydrierung in eine primäre Aminogruppe überführt werden kann.

Die Erfindung betrifft bevorzugt Cumarinderivate der Formel 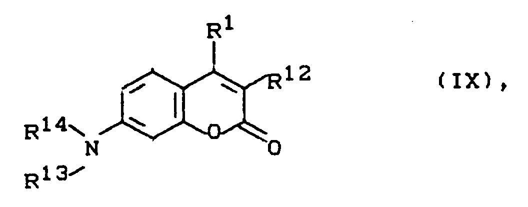 in der
- R¹: Wasserstoff oder Cyano bedeutet,
- R¹: für Phenyl oder für in 2-, 4- oder 5-Stellung gebundenes Thiazolyl steht, wobei Phenyl durch Carboxyl, C₁-C₄-Alkyl-carbonyloxy, Amino, -NH-C₁-C₄- Alkyl, -C₁-C₄-Alkyl-NH₂- C₁-C₄-Alkyl, Cyano, Fluor, Chlor oder Brom substituiert sein kann und wobei Thiazolyl durch Chlor, Cyano oder Carboxyl oder einen in 4- und 5-Stellung ankondensierten Benzolring, der seinerseits Carboxyl oder Amino tragen kann, substituiert sein kann,
- R¹³: Wasserstoff, Methyl oder Ethyl bedeutet und
- R¹⁴: für -C₁-C₄-Alkyl-OH, -C₁-C₄-Alkyl-NH₂ oder C₁-C₄-Alkyl-COOH steht,
wobei weiterhin R¹³ und R¹⁴ gemeinsam mit dem N-Atom, das sie substituieren, einen Morpholinring oder einen Piperazinring, der durch Methyl, Phenyl oder Methyl und Phenyl substituiert sein kann, bedeuten können.

Die Erfindung betrifft besonders bevorzugt Cumarinderivate der Formel 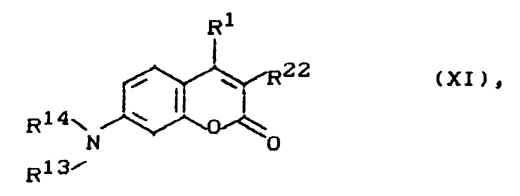 in der
- R¹, R¹³ und R¹⁴: den oben genannten Bedeutungsumfang haben und
- R: für Phenyl oder für in 2-Stellung gebundenes Thiazolyl steht, wobei Phenyl durch para-Carboxyl, para-Amino, para-NH-C₁-C₄-Alkyl, para-CH₂-NH₂, Cyano, Methyl oder Ethyl substituiert sein kann und wobei Thiazolyl durch Chlor, Cyano oder Carboxyl oder einen in 4- und 5-Stellung ankondensierten Benzolring, der seinerseits Carboxyl oder Amino tragen kann, substituiert sein kann.

Die Erfindung betrifft weiterhin ein Verfahren zur Herstellung von Cumarinderivaten der Formel 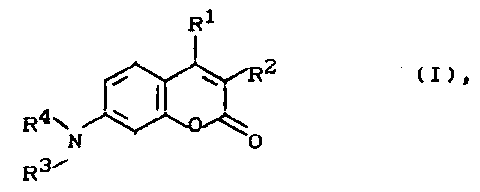 in der
- R¹: Wasserstoff oder Cyano bedeutet,
- R: für Phenyl oder für in 2-, 4- oder 5-Stellung gebundenes Thiazolyl steht, wobei Phenyl durch Cyano, Amino, -NH-C₁-C₄-Alkyl, -C₁-C₄-Alkyl-NH₂, -C₁-C₄-Alkyl-NH-C₁-C₄-Alkyl, Carboxyl, C₁-C₄- Alkoxy-carbonyl, C₁-C₄-Alkyl-carbonyloxy, Hydroxy, C₁-C₄-Alkylamino-carbonyl oder C₁-C₄-Alkylcarbonyl-amino substituiert ist und zusätzlich durch C₁-C₄- Alkyl, Fluor, Chlor oder Brom substituiert sein kann, und wobei Thiazolyl ein- oder zweifach durch Chlor, Cyano, Carboxyl oder C₁-C₄-Alkoxy-carbonyl substituiert ist, wobei bei zweifacher Substitution die beiden Substituenten verschieden sein können und wobei die 4- und die 5-Stellung gemeinsam einen ankondensierten Benzolring tragen können, der durch Carboxyl, Amino oder Hydroxy substituiert sein kann,
- R³: Wasserstoff, C₁-C₄-Alkyl oder C₁-C₄-Alkoxycarbonyl-C₁-C₄-alkyl bedeutet und
- R⁴: für C₁-C₄-Alkyl oder Phenylsulfonyl steht, wobei C₁-C₄-Alkyl durch Hydroxy, Amino, Carboxy oder C₁-C₄-Alkoxy-carbonyl substituiert sein kann und Phenyl ein- oder zweifach durch Chlor, Brom oder C₁-C₄-Alkyl substituiert sein kann,
wobei weiterhin R³ und R⁴ gemeinsam mit dem N-Atom, das sie substituieren, einen Morpholinring oder einen Piperazinring, der einen oder zwei Substituenten aus der Gruppe Methyl, Ethyl und Phenyl tragen kann, bedeuten können und
wobei weiterhin einer der Reste R, R³ und R⁴ eine primäre oder sekundäre Aminogruppe, die Hydroxylgruppe, die Carboxylgruppe oder die C₁-C₄-Alkoxy-carbonylgruppe trägt oder durch Verseifung von Cyano oder C₁-C₄-Alkoxy-carbonyl in die Carboxylgruppe, durch Verseifung von Acylamino oder Phenylsulfonylamino in eine Aminogruppe oder durch Hydrierung von Cyano in eine primäre Aminogruppe übergeführt werden kann,
das dadurch gekennzeichnet ist, daß man
a) ein m-Aminophenol und ein Formylessigsäurederivat der Formeln 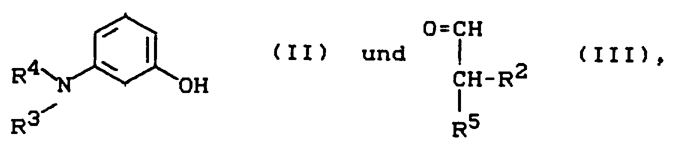 worin
   - R, R³ und R⁴: den obigen Bedeutungsumfang haben und
   - R⁵: Cyano, C₁-C₄-Alkoxy-carbonyl oder Carboxyl bedeutet,
   miteinander umsetzt oder
b) einen Salicylaldehyd und ein Essigsäurederivat der Formeln 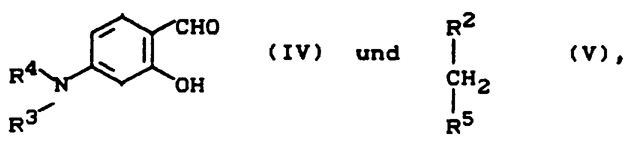 worin
   R, R³, R⁴ und R⁵ den obigen Bedeutungsumfang heben
   miteinander umsetzt,
   wobei im Falle von R⁵ = CN bei a) und b) zunächst ein Imino-Zwischenprodukt der Formel 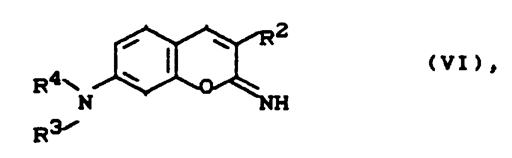
   worin R, R³ und R⁴ den obigen Bedeutungsumfang haben,
   entsteht und dieses Imino-Zwischenprodukt unter Abspaltung der Iminogruppe hydrolysiert wird, oder
c) für den Fall, daß R¹ Cyano bedeutet, das Imino-Zwischenprodukt gemäß b) oder das Cumarinderivat der Formel (I) mit Cyanidionen zum Imino-Cyano-Zwischenprodukt oder zum Cyano-Zwischenprodukt der Formeln 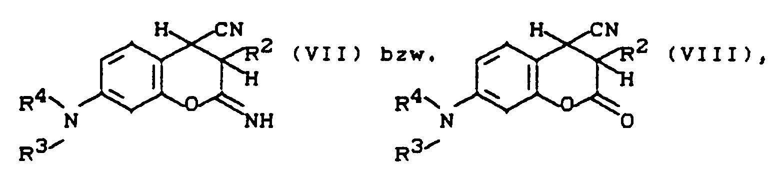 worin R, R³ und R⁴ den obigen Bedeutungsumfang haben,
   umsetzt und dies zum Cumarinderivat oxidiert und gegebenenfalls zusätzlich hydrolysiert.

Ein Teil der Verbindungen der Formeln (V), nämlich die Thiazolyl-essigsäurederivate der Formel 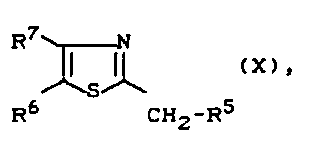
in der
- R⁶: Chlor oder Cyano und
- R⁷: Chlor bedeuten und
- R⁵: den oben genannten Bedeutungsumfang hat, sind neu.

Die Erfindung betrifft demnach auch die Zwischenprodukte der Formel (X).

Die Herstellung von Verbindungen der Formel (X) ist beispielhaft in den Ausführungsbeispielen beschrieben.

Die erfindungsgemäßen Cumarinderivate, bei denen einer der Reste R, R³ und R⁴ eine primäre oder sekundäre Aminogruppe, die Hydroxylgruppe, die Carboxylgruppe oder die C₁-C₄-Alkoxycarbonylgruppe trägt, sind über diese genannten Gruppen bindungsfähig an biologisch aktive Verbindungen und daher geeignet, biologisch aktive Verbindungen anzufärben. Solche angefärbten biologisch aktiven Verbindungen können beispielsweise in Immuno-assay-Methoden zur Detektion ihrer komplementären Verbindungen eingesetzt werden. Hierbei wird die Fluor-eszenzeigenschaft der zum Anfärben benutzten erfindungs-gemäßen Cumarinderivate ausgenutzt. Die biologisch aktiven Verbindungen und ihre Komplementärverbindungen sind beispielsweise das Paar Antigen/ Antikörper oder zwei zueinander komplementäre DNA-Stränge. Zur Vermeidung unübersichtlicher Mehrfachreaktionen zwischen den Cumarinen und biologisch aktiven Verbindungen sind die erfindungsgemäßen Cumarine darauf beschränkt, daß nur einer der Reste R, R³ und R⁴ eine der genannten bindungsfähigen Gruppen trägt.

Die Erfindung betrifft daher auch die Verwendung der erfindungsgemäßen Cumarinderivate der Formel (I) zum Anfärben von biologisch aktiven Verbindungen.

Cumarinderivate, die in 3-Position durch Phenyl oder Thiazolyl substituiert sind, in 4-Position durch Cyano substituiert sein können, aber in 7-Position anders substituiert sind als die erfindungsgemäßen Cumarinderivate sind aus EP 101 897 bekannt. Diese bekannten Cumarinderivate weisen nicht die zum Anfärben biologisch aktiver Verbindungen erforderlichen Gruppen auf; ihre Einsatzgebiete sind das Färben und Bedrucken von synthetischen und halbsynthetischen Materialien sowie die Verwendung als Energiewandler in Lichtsammelsystemen.

C₁-C₄-Alkyl bzw. C₁-C₄-Alkoxy können geradkettig oder verzweigt sein und sind beispielsweise Methyl, Ethyl, Propyl, Isopropyl, Butyl, Isobutyl,Methoxy, Ethoxy, Propoxy, Isopropoxy, Butoxy, Isobutoxy. In bevorzugter Weise seien Methyl, Ethyl, Methoxy und Ethoxy genannt. Bevorzugtes C₁-C₄-Alkoxy-carbonyl ist Methoxy-carbonyl und Ethoxy-carbonyl.

Die Herstellung der erfindungsgemäßen Cumarinderivate gemäß Herstellungsvariante b) im Sinne einer Knoeve-nagel-Kondensation sei beispielsweise durch das folgende Formelschema exemplifiziert:

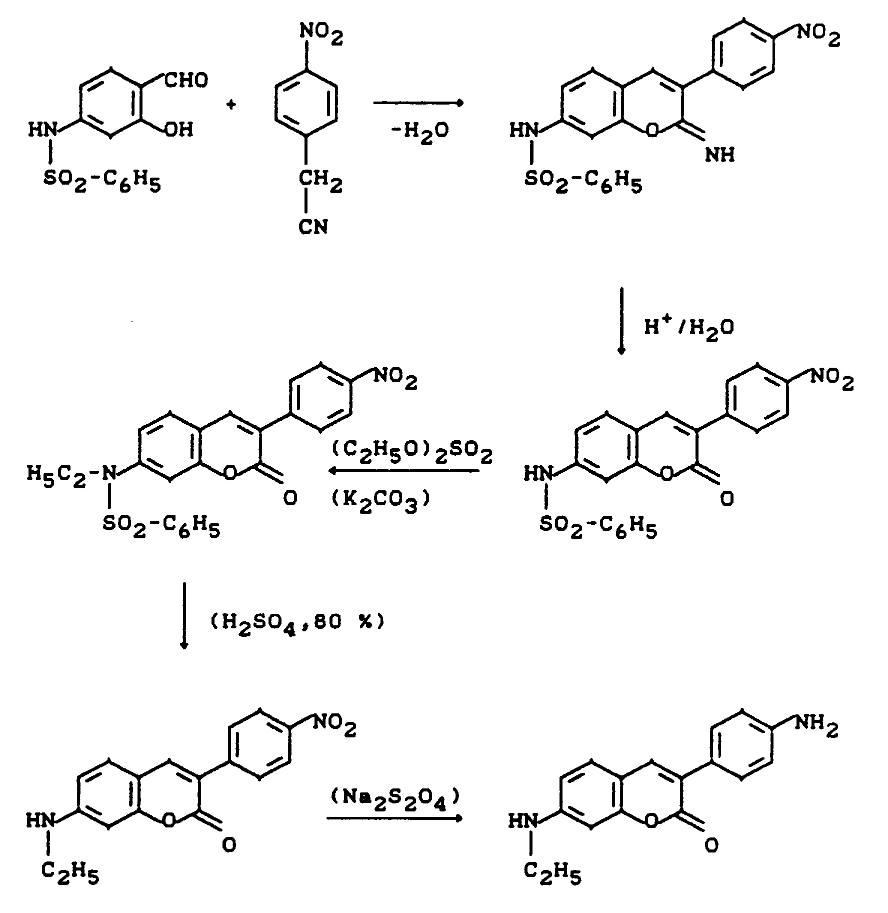

Hierbei wird ein Salicylaldehyd, der entsprechend Formel (IV) in para-Stellung zur Aldehydgruppe substituiert ist, mit einem Essigsäurederivat, das entsprechend Formel (V) substituiert ist, unter Wasseraustritt kondensiert, wobei für den Fall, daß es sich um das Essigsäurenitril handelt zunächst das Imino-Zwischenprodukt, wie im obigen Formelschema dargestellt, entsteht, welches durch Behandlung mit verdünnten Säuren, beispielsweise mit verdünnter Salzsäure, in das zugehörige Cumarinderivat übergeführt werden kann. Wenn das angestrebte Cumarinderivat Estergruppen enthält, wird eine solche Behandlung mit verdünnten Säuren in Gegenwart von größeren Mengen an Alkohol (Methanol, Ethanol, Propanol) bei Temperaturen im Bereich von 20-45°C durchgeführt, wobei die Umwandlung des Imino-Zwischenprodukts unter Erhaltung der Estergruppen erreicht wird. Sofern aus ursprünglich im Cumarinderivat vorhandenden Estergruppen die freien Carboxylgruppen gebildet werden sollen, kann zur Umwandlung des Imino-Zwischenprodukts mit höher konzentrierten wässrigen Säuren bei höheren Temperaturen, etwa im Bereich von 70-100°C, gearbeitet werden, wobei gleichzeitig eine Verseifung zur Carboxylgruppe stattfindet.

An den Resten R und R³ kann beispielsweise eine erfindungsgemäß erforderliche funktionelle Gruppe aus dem Bereich der primären und sekundären Aminogruppe der Hydroxylgruppe, der Carboxylgruppe oder der C₁-C₄-Alkoxy-carbonylgruppe durch weitere Umsetzungen entwickelt werden. Im Beispiel des obigen Formelschemas kann die Phenylsulfonylgruppe als Rest R⁴ durch Verseifung, etwa mit 80 %iger Schwefelsäure, abgespalten werden. Zuvor kann jedoch, ebenfalls im Sinne des obigen Formelschemas, das genannte N-Atom selektiv einfach alkyliert werden, beispielsweise mit Hilfe von Diethylsulfat in Gegenwart von schwachen Akalien; wird erst danach die Phenylsulfonylgruppe hydrolytisch abgespalten, bilden R³ und R⁴ gemeinsam mit dem N-Atom, das sie Substituieren, eine sekundäre Aminogruppe. Noch weiterhin kann, wie ebenfalls im Formelschema dargestellt, die innerhalb des Restes R vorhandene Nitrogruppe zur primären Aminogruppe reduziert werden; hierzu kann beispielsweise Natriumdithionit als Reduktionsmittel eingesetzt werden.

Da freie primäre Aminogruppen mit Salicylaldehyden zu Schiff'achen Basen reagieren, ist es erforderlich, solche primären Aminogruppen zu schützen, beispielsweise durch eine Acylgruppe oder die Phenylsulfonylgruppe. Eine solche Schutzgruppe kann in bereits oben geschilderter Weise durch Hydrolyse wieder abgespalten werden. Im Sinne der obigen Ausführungen kann selbstverständlich eine freie primäre Aminogruppe auch durch Reduktion (Hydrierung) aus einer Nitrogruppe oder einer Cyanogruppe entwickelt werden. Als Reduktionsmittel kommen Natriumdithionit, Zinn(II)-Salze oder katalytisch angeregter Wasserstoff in Frage.

Für den Fall der Einführung und Hydrierung einer Cyanogruppe ergibt sich folgender Reaktionsablauf:

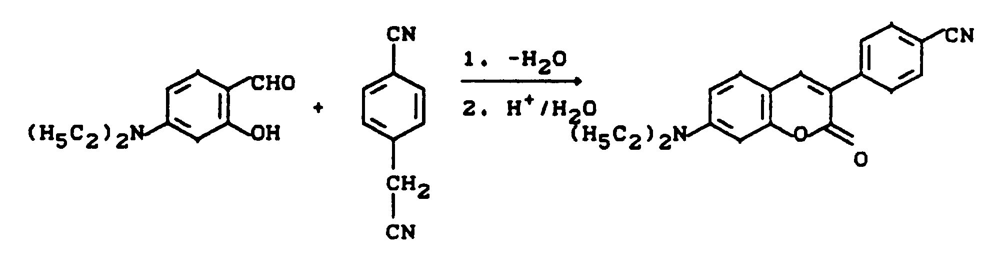

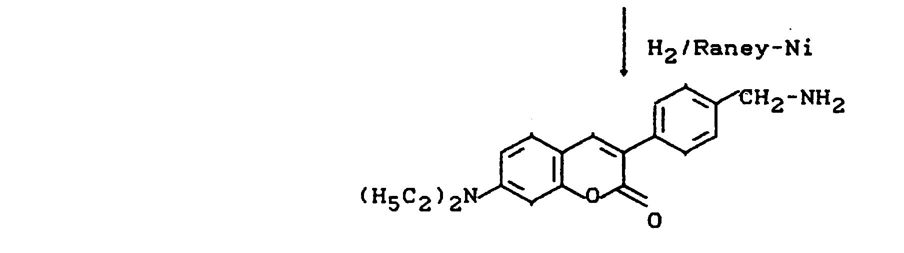

Gemäß einer weiteren Herstellung kann nach a) von einem Aminophenol der Formel (II) und einem Formylessigsäurederivat der Formel (III) ausgegangen werden. Diese beiden Ausgangsstoffe werden in ähnlicher Weise, wie weiter oben für b) geschildert wurde, unter Wasseraustritt kondensiert. Für den Fall, daß R⁵ Methoxycarbonyl oder Ethoxycarbonyl bedeutet, wird zusätzlich Methanol bzw. Ethanol abgespalten. Für den Fall, daß R⁵ Cyano bedeutet, entsteht ebenfalls wie bei der Reaktionsfolge nach b) zunächst das Imino-Zwischenprodukt der Formel (VI), welches mit verdünnter Säure zum Cumarinderivat umgewandelt wird.

Die Funktionalitäten, durch die die erfindungsgemäßen Cumarinderivate ausgezeichnet sind, nämlich primäre oder sekundäre Aminogruppen, Hydroxylgruppen, Carboxylgruppen oder C₁-C₄-Alkoxycarbonylgruppen bzw. die genannten Gruppen, aus denen solche funktionellen Gruppen entwickelt werden können (Cyanogruppe oder durch Acyl oder Phenylsulfonyl geschützte Aminogruppen) können im Rahmen der geschilderten Herstellungsmöglichkeiten entweder über die zur Herstellung eingesetzten Salicylaldehyde oder die eingesetzten Aminophenole oder über die genannten Essigsäurederivate eingeführt werden. Im ersteren Falle handelt es sich um in 7-Position des Cumarinderivats angeordnete funktionelle Gruppen; im letzteren Fall handelt es sich um in 3-Position der Cumarinderivate angebrachte funktionelle Gruppen.

Die erfindungsgemäßen Cumarinderivate können weiterhin in 4-Position durch Umsetzung mit Cyanidionen und anschließende Oxidation mit einer Cyanogruppe versehen werden. Dies ergibt eine bathochrome Verschiebung der Wellenlänge der Lichtabsorption solcher Cumarinderivate. Diese Umsetzung kann sowohl an den Cumarinderivaten der Formel (I), in denen R¹ Wasserstoff bedeutet, als auch an dem Imino-Zwischenprodukt der Formel (VI) vorgenommen werden. Die Umsetzung nimmt man in einem für das Cumarinderivat oder Imino-Zwischenprodukt geeigneten Lösungsmittel, beispielsweise Dimethylformamid, vor. Die Cyanidionen werden beispielsweise in Form einer wässrigen Natrium- oder Kaliumcyanidlösung zugesetzt. Die nachfolgende Oxidation kann beispielsweise mit Bleitetraacetat, Wasserstoffperoxid, Brom, Persulfaten oder anderen dem Fachmann bekannten Oxidationsmitteln vorgenommen werden.

Die Einführung der Cyanogruppe nach c) läßt sich formelmäßig beispielsweise wie folgt darstellen:

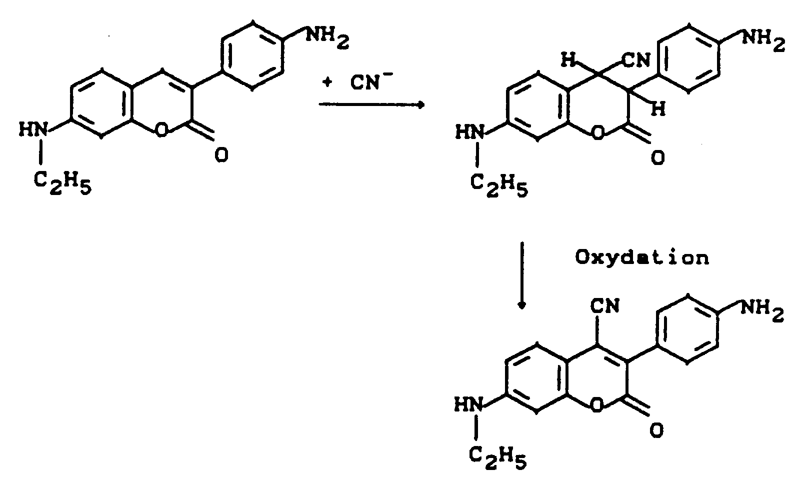

Die Umsetzung mit Cyanidionen kann im Falle von Carboxylgruppen enthaltenden Cumarinen in Form von deren Natriumsalz vorgenommen werden, welches beispielsweise in Dimethylformamid ausreichend löslich ist.

Die für die Herstellungsvariante a) erforderlichen Aminophenole können durch Reaktion von Resorcin mit einem primären oder sekundären Amin unter Wasserabspaltung gewonnen werden, wie es beispielhaft durch die folgende Formelgleichung dargestellt wird.

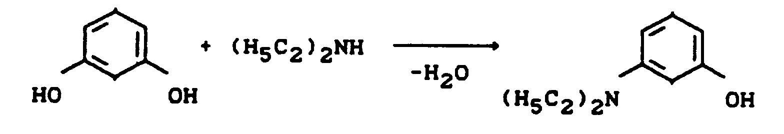

Solche m-Aminophenole können jedoch auch durch selektive N-Alkylierung von m-Aminophenolen mit Alkylhalogeniden in Anwesenheit eines säurebindenden Mittels, beispielsweise Calciumcarbonat, in grundsätzlich bekannter Weise gewonnen werden, wie es beispielhaft durch folgende Formelgleichung dargestellt wird:

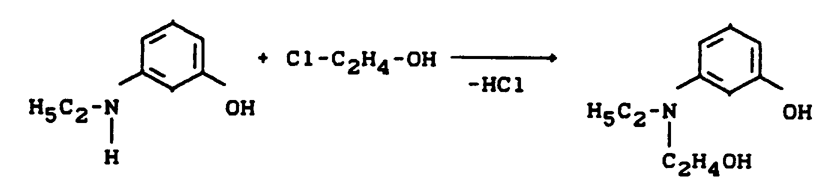

Die für die Herstellungsvariante b) erforderlichen Salicylaldehyde können durch Vilemeyer-Reaktion aus den zugrundeliegenden m-Aminophenolen, Phosphoroxichlorid und einem Formamid, beispielsweise Dimethylformamid, gewonnen werden, wie es beispielhaft durch folgende Formelgleichung dargestellt wird:

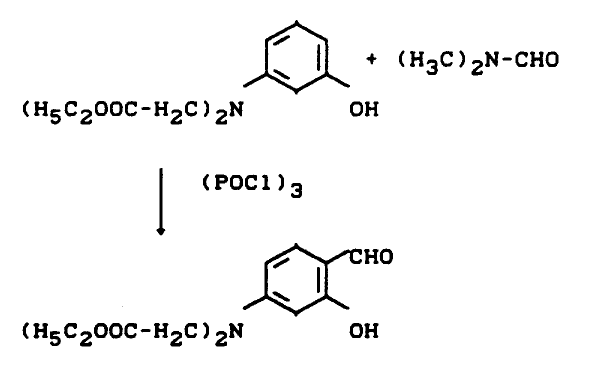

### Beispiele:

### Beispiel 1:

10 g (0,032 Mol) 7-Ethylamino-3-(4-nitrophenyl)-cumarin wurden in 70 ml DMF auf 100°C erhitzt, und unter gutem Rühren wurde eine Aufschlämmung von 20 g (0,11 Mol) Natriumdithionit in 50 ml Wasser hinzugetropft. Nach 25 min wurde das Lösungsmittel abdestilliert und der Rückstand mit 100 ml Wasser ausgerührt.

Nach Absaugen wurde dieser Rückstand mit 40 ml konzentrierter Salzsäure und 80 ml Ethanol zur Zerstörung entstandener Sulfamidsäuren 2 Stunden unter Rückfluß erhitzt. Es wurde mit Natriumhydrogencarbonat neutralisiert und abgesaugt.

Ausbeute an 7-Ethylamino-3-(4-aminophenyl)-cumarin: 8,1 g (91 %) Schmelzpunkt: 263°C,

### Beispiel 2:

In gleicher Weise wie in Beispiel 1 erhielt man 7-Methylamino-3-(4-aminophenyl)-cumarin.

### Beispiel 3:

13,6 g (0,07 Mol) 4-Diethylamino-salicylaldehyd wurden mit 10 g (0,07 Mol) α-Cyano-toluolnitril, 5 ml Piperidin und 3 ml Essigsäure in 200 ml Ethanol unter Rückfluß erhitzt. Nach dem Abkühlen wurde abgesaugt und der Rückstand mit 100 ml halbkonz. Salzsäure 1 Stunde zum Rückfluß erhitzt. Nach dem Neutralisieren mit Natronlauge wurde das Produkt mit Wasser gewaschen und abgesaugt.

Ausbeute an 7-Diethylamino-3-(4-cyanophenyl)-cumarin 15,7 g (70 %).

### Beispiel 4:

15 g (0,047 Mol) 7-Diethylamino-3-(4-cyanophenyl)-cumarin wurden in 100 ml Ethanol und 30 ml flüssigem Ammoniak bei 100 bar Wasserstoffdruck und 70°C mit Raney-Nickel als Katalysator hydriert. Nach Beendigung der Reaktion wurde das Lösungsmittel im Rotationsverdampfer abdestilliert, der Rückstand mit DMF aufgekocht und filtriert. Nach Abdestillieren des DMF wurde der Rückstand aus Toluol unter Zugabe von Tonsil umgelöst. Bei 0°C fielen 4,9 g 7-Diethylamino-3-(4-methylaminophenyl)-cumarin aus (32 %), Schmelzpunkt: 118° C.

### Beispiel 5:

5 g (0,014 Mol) 7-Morpholino-3-(4-nitrophenyl)-cumarin wurden in 20 ml DMF auf 60°C erhitzt, und eine Aufschlämmung von 10 g (0,055 Mol) Natriumdithionit in 20 ml Wasser wurde hinzugetropft. Nach 1 Stunde wurden die Lösungsmittel im Vakuum abdestilliert und der Rückstand 2 Stunden lang mit einer Mischung aus 20 ml Ethanol und 30 ml konzentrierter Salzsäure aufgekocht. Nach Neutralisation mit Natronlauge wurde das ausgefallene Produkt abgesaugt, bei 50°C getrocknet und anschließend aus siedendem Chlorbenzol unter Zusatz von Tonsil umkristallisiert.

Ausbeute an 7-Morpholino-3-(4-aminophenyl)-cumarin: 2,6 g (57 %), Schmelzpunkt: 211- 216°C.

### Beispiel 6:

Aus 4-(N-Ethyl-N-β-hydroxyethylamino)-salicylaldehyd und p-Nitrophenyl-essigsäurenitril erhielt man 7-(N-Ethyl-N-β-hydroxyethylamino)-3-(4-nitrophenyl)-cumarin in 80 % der theoretischen Ausbeute und daraus weiter analog zu Beispiel 5 7-(N-Ethyl-N-β-hydroxyethylamino)-3-(4'-aminophenyl)-cumarin in 45 % der theoretischen Ausbeute, Schmelzpunkt 155° C.

### Beispiel 7:

10,8 g (0,05 Mol) 4-Chlor-5-cyano-thiazolyl-2-essigsäuremethylester, 10,46 g (0,05 Mol) 4-(N-2-Hydroxyethyl-N-ethyl-amino)-salicylaldehyd, 0,5 ml Piperidin und 0,3 ml Essigsäure wurden in 150 ml Ethanol 4 Stunden unter Rückfluß erhitzt. Das beim Abkühlen ausfallende Produkt wurde aus siedendem Chlorbenzol unter Zusatz von Bleicherde (Tonsil) umkristallisiert. Man erhielt 10,0 g (53 %) 7-(N-Ethyl-N-β-hydroxyethylamino)-3-(4'-chlor-5' cyano-thiazol-2'-yl)-cumarin vom Schmelzpunkt 226° C.

### Beispiel 8:

Analog zu Beispiel 7 erhielt man unter Verwendung von 4,5-Dichlor-thiazolyl-2-essigsäureethylester das 7-(N- Ethyl-N-β-hydroxyethylamino)-3-(4',5'-dichlor-thiazol-2'-yl)-cumarin vom Schmelzpunkt 230°C in 48 % der theoretischen Ausbeute.

### Beispiel 9:

Analog zu Beispiel 7 erhielt man unter Verwendung von 4,5-Benzothiazolyl-2-essigsäuremethylester das 7-(N- Ethyl-N-β-hydroxyethylamino)-3-(4',5'-benzathiazol-2'-yl)-cumarin vom Schmelzpunkt 221°C in 63 % der theoretischen Ausbeute.

### Beispiel 10:

Durch Anlagerung von Cyanid und anschließende Oxidation erhielt man aus dem Cumarin von Beispiel 7 das 7-(N-Ethyl-N-β- hydroxyethylamino)-3-(4'-chlor-5'-cyano-thiazol-2'-yl)- 4-cyano-cumarin vom Schmelzpunkt 292°C in 64 % der theoretischen Ausbeute.

### Beispiel 11:

Analog Beispiel 10 erhielt man aus dem Cumarin von Beispiel 9 das 7-(N-Ethyl-N-β-hydroxyethylamino)-3-(4',5'-benzothiazol-2'-yl)-4-cyano-cumarin vom Schmelzpunkt 263°C in 26 % der theoretischen Ausbeute.

### Beispiel 12:

27,7 g (0,1 Mol) BESA (4-(Benzolsulfonamido)-salicylaldehyd), 17,5 g (0,1 Mol) 4-Cyanomethyl-benzoesäuremethylester, 2,5 ml Piperidin und 1,7 ml Eisessig in 200 ml Ethanol wurden 3 Stunden unter Rückfluß erhitzt. Nach Erkalten wurde abgesaugt und das ausgefallene Material mit 100 ml Methanol und 100 ml konz. Salzsäure 2 Stunden unter Rückfluß erhitzt. Es fielen 25,3 g (58 %) 7-Benzolsulfonamino-3-(4'-methylcarboxyphenyl)-cumarin aus, die ohne weitere Vorreinigung für Beispiel 17 eingesetzt werden konnten.

### Beispiel 13:

35 g (0,08 Mol) der Verbindung aus Beispiel 12, 30 g (0,22 Mol) Kaliumcarbonat, 30,8 g (0,2 Mol) Diethylsulfat und 200 ml DMF wurden 48 h unter gutem Rühren auf 50°C erhitzt. Danach wurde das Lösungsmittel abdestilliert und der Rückstand mit Wasser ausgerührt. Nach Absaugen und Trocknen erhielt man 35 g 7-(N-Ethyl-N-benzolsulfonylamino)-3-(4'-methyl-carboxyphenyl)-cumarin (83 %).

### Beispiel 14:

35 g (0,075 Mol) 7-(N-Ethyl-N-benzolsulfonylamino)-3-(4'-methylcarboxyphenyl)-cumarin wurden in 65 ml 80 %iger Schwefelsäure 3 Stunden lang auf 100°C erwärmt. Zur Aufarbeitung wurde vorsichtig in 400 ml Eiswasser gegeben und mit Natronlauge auf pH 6,5 gebracht. Nach Absaugen wurde mit Wasser gewaschen. Ausbeute: 16 g (68 %) 7-(N-Ethyl)-3-(4'-carboxyphenyl)-cumarin.

### Beispiel 15:

Analog zu Beispiel 8 erhielt man unter Verwendung von 4-(N,N-Bis-[ethoxycarbonylmethyl]-amino)-salicylaldehyd das 7-(N,N-Bis-[ethoxycarbonylmethyl]-amino)-3-(4',5'-dichlor-thiazol-2'-yl)-cumarin vom Schmelzpunkt 190°C in 62 % der theoretischen Ausbeute.

### Beispiel 16:

3,5 g (0,0072 Mol) des Cumarins aus Beispiel 15 in 50 ml DMF wurden bei 40°C mit einer Lösung von 1 g (0,015 Mol) Kaliumcyanid in 10 ml Wasser versetzt und drei Stunden bei dieser Temperatur gerührt. Man kühlte auf 0-5°C ab und gab 4,0 g (0,009 Mol) Bleitetraacetat in 10 ml DMF hinzu. Nach 2 Stunden wurde das ausgefallene Material abgesaugt, mit Methanol gewaschen und aus 200 ml siedendem Toluol unter Zusatz von Bleicherde (Tonsil) umkristallisiert, Ausbeute: 1,6 g (43 %) 7-(N,N-Bis-[ethoxycarbonylmethyl]-amino)-3-(4',5'-dichlor-thiazol-2'-yl)-4-cyano-cumarin vom Schmelzpunkt 185° C.

### Beispiel 17:

5,4 g (0,02 Mol) 4,5-Bis(ethoxycarbonyl)-2-thiazolylessigsäurenitril, 4,2 g (0,02 Mol) 4-Morpholinosalicylaldehyd, 0,5 ml Piperidin und 0,3 ml Essigsäure wurden in 100 ml Ethanol unter Rückfluß 3 Stunden lang erhitzt. Das nach dem Abkühlen ausfallende Zwischenprodukt (Imin) wurde bei 45°C in 80 ml Ethanol, 10 ml konzentrierter Salzsäure und 10 ml Wasser 4 Stunden lang gerührt. Nach dem Neutralisieren mit 20 %iger Natronlauge wurde aus Toluol unter Zusatz von Bleicherde (Tonsil) umkristallisiert.

Man erhielt 7,4 g (80 %) 7-(N-Morpholino)-3-(4',5'-bis-[ethoxycarbonyl]-thiazol-2'-yl)-cumarin vom Schmelzpunkt 265° C.

### Beispiel 18:

5,6 g (0,02 Mol) 4-[N-Ethyl-N-(4-ethylbutyrat)-amino]-salicylaldehyd, 3,5 g Benzthiazolylessigsäurenitril, 0,5 ml Piperidin und 0,3 ml Essigsäure wurden 3 Stunden unter Rückfluß in 100 ml Ethanol erhitzt. Nach Abdestillieren des Ethanols wurde weitere 2 Stunden mit 100 ml halbkonzenzrierter Salzsäure zum Sieden erhitzt. Nach dem Abkühlen wurde mit Natronlauge auf pH 6,5 eingestellt, abgesaugt und aus siedender Essigsäure unter Zusatz von Aktivkohle umkristallisiert. Man erhielt 5,5 g 7-[N-Ethyl-N-(carboxy-tetramethylen)-amino]-3-(4',5'-benzothiazol-2'-yl)-cumarin vom Schmelzpunkt 218°C in 67 % der theoretischen Ausbeute.

### Beispiel 19:

Analog zur beschriebenen Verfahrensweise erhielt man die Verbindung

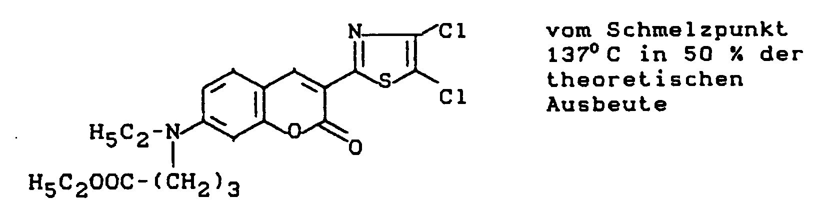

### Beispiel 20:

Analog zur beschriebenen Verfahrensweise erhielt man die Verbindung

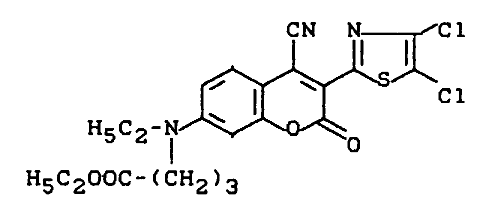

### Beispiel 21:

Analog zur beschriebenen Verfahrensweise erhielt man die Verbindung

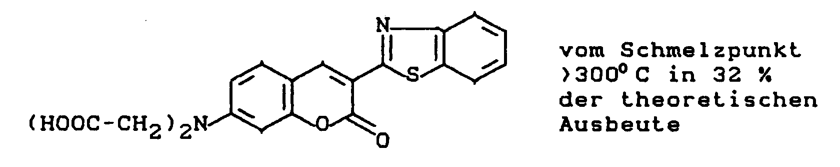

### Beispiel 22:

Analog zur beschriebenen Verfahrensweise erhielt man die Verbindung

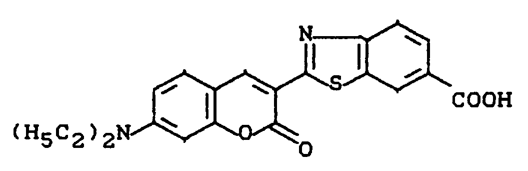

### Beispiel 23:

Analog zur beschriebenen Verfahrensweise erhielt man die Verbindung

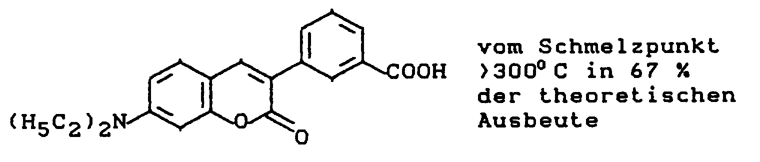

### Beispiele 24 - 32:

Synthese der verwendeten m-Aminophenole

Die verwendeten m-Aminophenole konnten durch zwei Verfahren gewonnen werden:
a) Reaktion von Resorcin mit einem primären oder sekundären Amin unter Wasserabspaltung, wie in DE-OS 15 43 368 beschrieben.
b) Selektive N-Alkylierung von m-Aminophenolen mit Alkylhalogeniden in Anwesenheit eines säurebindenden Mittels (z.B. Calciumcarbonat). Vorzugsweise wurden alkylierendes Agens und säurebindendes Mittel in 20-50 %igem Überschuß eingesetzt. Dabei erfolgte selektiv eine N-Alkylierung.

### Beispiel 24:

110 g (1,0 Mol) Resorcin, 89 g (1,0 Mol) N-(2-Hydroxy-ethyl)-ethylamin und 6,2 g (0,1 Mol) Borsäure wurden auf 180 - 200°C erhitzt und das entstehende Wasser über einen Zeitraum von ca. 8 Std. abdestilliert. Nach Abkühlen auf 60°C wurden 130 ml Methanol zugegeben und der gebildete Borsäuremethylester abdestilliert. Im Hochvakuum wurden dann zunächst nicht umgesetztes Amin und Resorcin, anschließend das Produkt abdestilliert.

Siedepunkt: 191°C (3 mbar) Ausbeute an 3-(N-2-Hydroxy-ethyl-N-ethyl)aminophenol: 40 %
Reinheit laut GC: 80 %
Diese Verbindung konnte auch nach Methode b) aus N-Ethyl-m-aminophenol und 2-Chlorethanol hergestellt werden.

### Beispiel 25:

149,1 g (1,0 Mol) 3-Ethylaminophenol (92 % Reinheit), 120 g (1,2 Mol) Calciumcarbonat (gemahlen) und 500 ml DMF wurden auf 120°C erwärmt, und während 4-5 Stunden langsam 80,5 g (1,0 Mol) 2-Chlorethanol hinzugetropft. Es wurde noch 12 h bei 125°C nachgerührt und dann das Lösungsmittel abdestilliert. Der Rückstand wurde mit 300 - 500 ml Wasser versetzt und filtriert. Nach dreimaligem Ausschütteln mit Chloroform (je 150 ml) wurden die vereinigten organischen Phasen abdestilliert. Man erhielt 67 g Produkt (Reinheit 95 %).
Kp.: (0,15) mbar: 180°C

Um die alkoholische OH-Gruppe bei der nachfolgenden Vilsmeyer-Reaktion zu schützen, wurde eine Acetylierung wie folgt durchgeführt:

95 g (0,5 Mol) 3-(N-2-Hydroxyethyl-N-ethyl)aminophenol, 113 g (1,1 Mol) Essigsäureanhydrid und 87 g (1,0 Mol) Pyridin wurden 3 Stunden lang unter Rückfluß erhitzt. Zur Aufarbeitung wurde auf 300 g Eis gegeben, mit Natriumhydrogencarbonat neutral gestellt und mehrfach mit Chloroform ausgeschüttelt. Nach Trocknung über Natriumsulfat wurden die vereinigten organischen Phasen im Vakuum destilliert. Bei 0,3 mbar und 166 - 170°C gingen 78,2 g (56 %) 3-(N-2-Acetoxy-ethyl-N-ethyl)amino-acetoxyphenol über.

### Beispiel 26:

14,9 g (0,1 Mol) 3-Ethylaminophenol (92 X Reinheit) wurden in 35 - 50 ml DMF mit 11 g (0,11 Mol) feingemahlenem Calciumcarbonat auf 65°C erhitzt. Innerhalb von 30 min wurden 16,7 g (0,1 Mol) Bromessigsäureethylester hinzugetropft. Es wurde 1 Stunde bei 80°C nachgerührt. Nach Abdampfen des Lösungsmittels wurde der Rückstand mit 100 ml Wasser und 50 ml Chloroform versetzt und filtriert. Die wässrige Phase wurde erneut mit Chlorform extrahiert. Die vereinigten organischen Phasen wurden über Natriumsulfat getrocknet und danach im Vakuum destilliert.
Bei 0,15 mbar und 167-171°C gingen 16,3 g (73 %) Produkt über.

In analoger Weise wurden dargestellt (Beispiele 27-32)

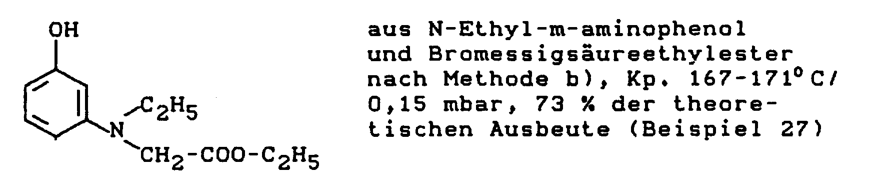

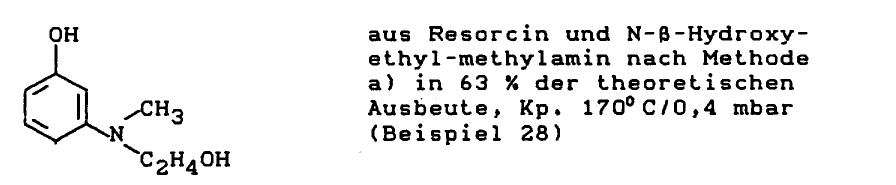

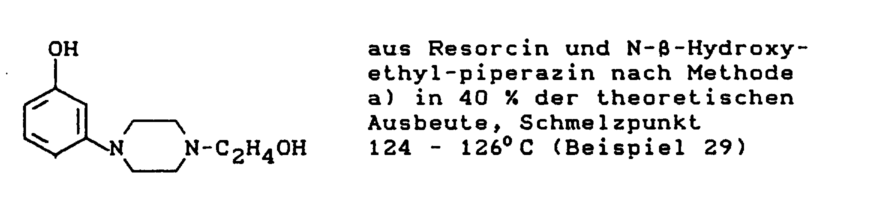

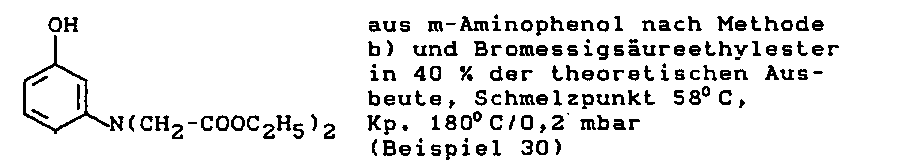

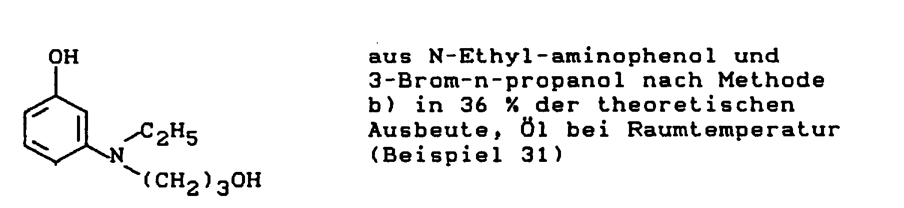

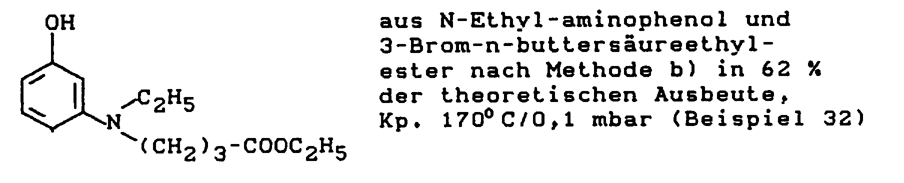

### Beispiele 33 - 38:

Synthese der verwendeten 4-Amino-salicylaldehyde durch Vilsmeyer-Reaktion aus den zugrundeliegenden 3-Aminophenolen. Hydroxylgruppen wurden durch Acetylierung und Carboxylgruppen durch Veresterung geschützt.

### Beispiel 33:

16,9 g (0,06 Mol) 3-(N,N-Diessigsäureethylester)-aminophenol wurden in 40 ml DMF gelöst und 12 g (7,4 ml, 0,078 Mol) Phosphoroxychlorid langsam zugetropft, so daß die Innentemperatur 20°C nicht überschritt. Zur Aufarbeitung wurde auf Eis gegeben, mit Natronlauge neutralisiert und mit Chloroform ausgeschüttelt. Nach Trocknen der vereinigten organischen Phasen wurde das Chloroform abdestilliert. Der Rückstand wurde mit Toluol und Tonsil aufgekocht. Abdampfen des Lösungsmittels lieferte 12,6 g (68 %) Produkt von Schmelzpunkt 70°C.

### Beispiel 34:

Zu 18 g (0,068 Mol) 3-(N-2-Acetoxyethyl-N-ethyl-amino)-acetoxyphenol in 20 ml DMF wurden 12,34 g (7,8 ml, 0,08 Mol) Phosphoroxychlorid hinzugetropft, so daß die Innentemperatur 20°C nicht überschritt. Nach 1 Stunde bei dieser Temperatur wurde 5 h lang auf 60°C erwärmt. Zur Aufarbeitung wurde vorsichtig auf 200 g Eis gegeben, mit Natronlauge neutralisiert und mehrfach mit Chloroform ausgeschüttelt. Nach Trocknen der vereinigten organischen Phasen wurde das Chloroform am Rotationsverdampfer entfernt und das zurückbleibende Öl 6 Stunden lang mit halbkonzentrierter Salzsäure zur Schutzgruppenabspaltung gerührt. Nach Neutralisation, Ausschütteln mit Chloroform und Trocknen sowie Abdampfen des Lösungsmittels wurde der Rückstand mit Toluol und Tonsil aufgekocht. Nach Filtration und Entfernen des Toluols blieben 7,1 g (50 %) 4-(N-2-Hydroxyethyl-N-ethyl-amino)-salicylaldehyd vom Schmelzpunkt 60°C zurück.

In analoger Weise wurden dargestellt (Beispiele 35-38)

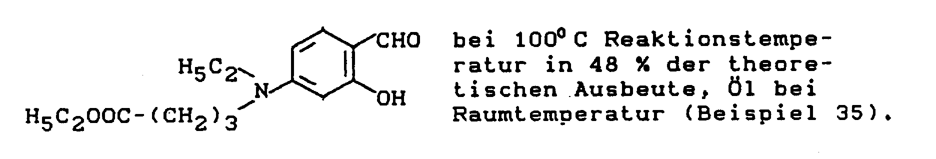

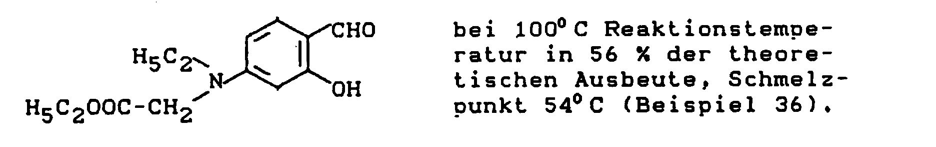

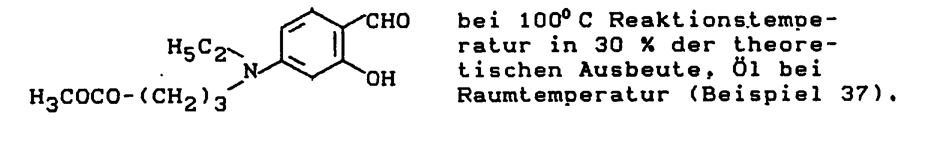

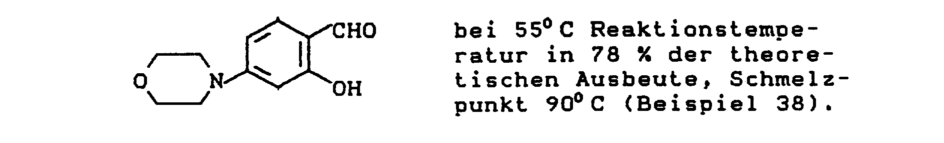

### Beispiel 39

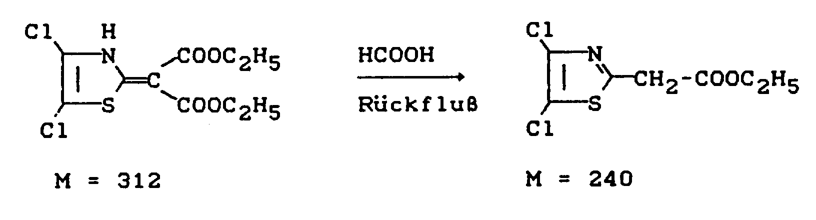

656 g (2,1 Mol) 4,5-Dichlor-2-thiazolylmalonsäurediethylester und 3 Liter reine Ameisensäure wurden 7 Stunden unter Rückfluß gerührt, Nach dem Eindampfen im Rotationsverdampfer (509 g; quant.) wurde an der Ölpumpe destilliert. Ausbeute 463 g (92 % der Theorie) 4,5-Dichlor-2-thiazolylessigsäureethylester. Siedepunkt 120°C/0,1 mbar. Schmelzpunkt unter 50°C (aus wenig Petrolether umkristallisierbar).

### Analog wurde erhalten:

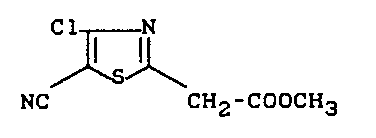

4-Chlor-5-cyan-2-thiazolylessigsäuremethylester. Schmelzpunkt 63°C.

### Beispiel 40:

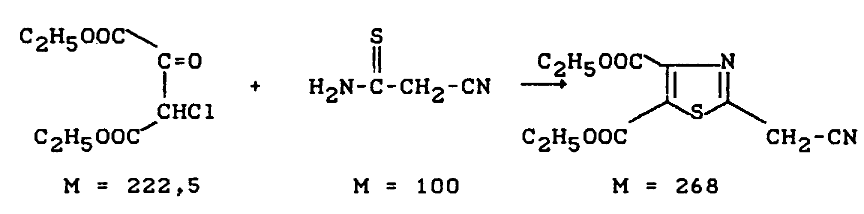

40 g (0,4 Mol) Cyanessigsäurethioamid wurden in 200 ml Dimethylformamid gelöst. Nach Zugabe von 111 g (0,5 Mol) Chloroxalessigsäurediethylester wurde 4 Tage bei Raumtemperatur gerührt. Anschließend wurde in etwa 2,5 Liter Eiswaser gegossen, mit Dichlormethan aufgenommen und die Dichlormethan-Phase eingeengt. Fraktionierte Destillation im Bereich von 150-230°C/1,5-2 mbar lieferte 11,6 g Rohprodukt, das aus Hexan-Toluol (3:1) umkristallisiert wurde. Ausbeute: 7,6 g 4,5-Bis(ethoxycarbonyl)-2-thiazolylessigsäurenitril von einer GC-Reinheit von 99,4 %. Schmelzpunkt 75,7-77°C:

In 100 ml Nitrobenzol wurden 14,66 g (0,11 Mol) wasserfreies Aluminiumchlorid vorgelegt. Dazu gab man 16,5 g (0,1 Mol) 3-N,N-Diethylwmino-phenol und 19 g (0,1 Mol) α-(Hydroxymethylen)-4-nitrophenyl-acetonitril (Herstellung: B. Boose et. al., Bull. Soc. Chim. Belg., 1988, [97], 267-70). Die Mischung wurde 2 Stunden bei 100°C gerührt, dabei schied sich ein Niederschlag ab. Man erhöhte nun die Temperatur auf 130° und rührte weitere 1,5 Stunden. Nach dem Abkühlen auf etwa 80° wurden 100 ml Isopropanol und 50 ml Wasser zugegeben. Anschließend tropfte man bei 80° 30 ml 37 %ige Salzsäure zu. Nach dem Abkühlen und Absaugen des gebildeten Niederschlages erhielt man 29 g (85,8 % d.Th.) 3-(4-Nitrophenyl)-7-diethylaminocumarin als gelbe Kristalle, die nach dem Umlösen aus Acetonitril bei 162-3°C schmelzen.

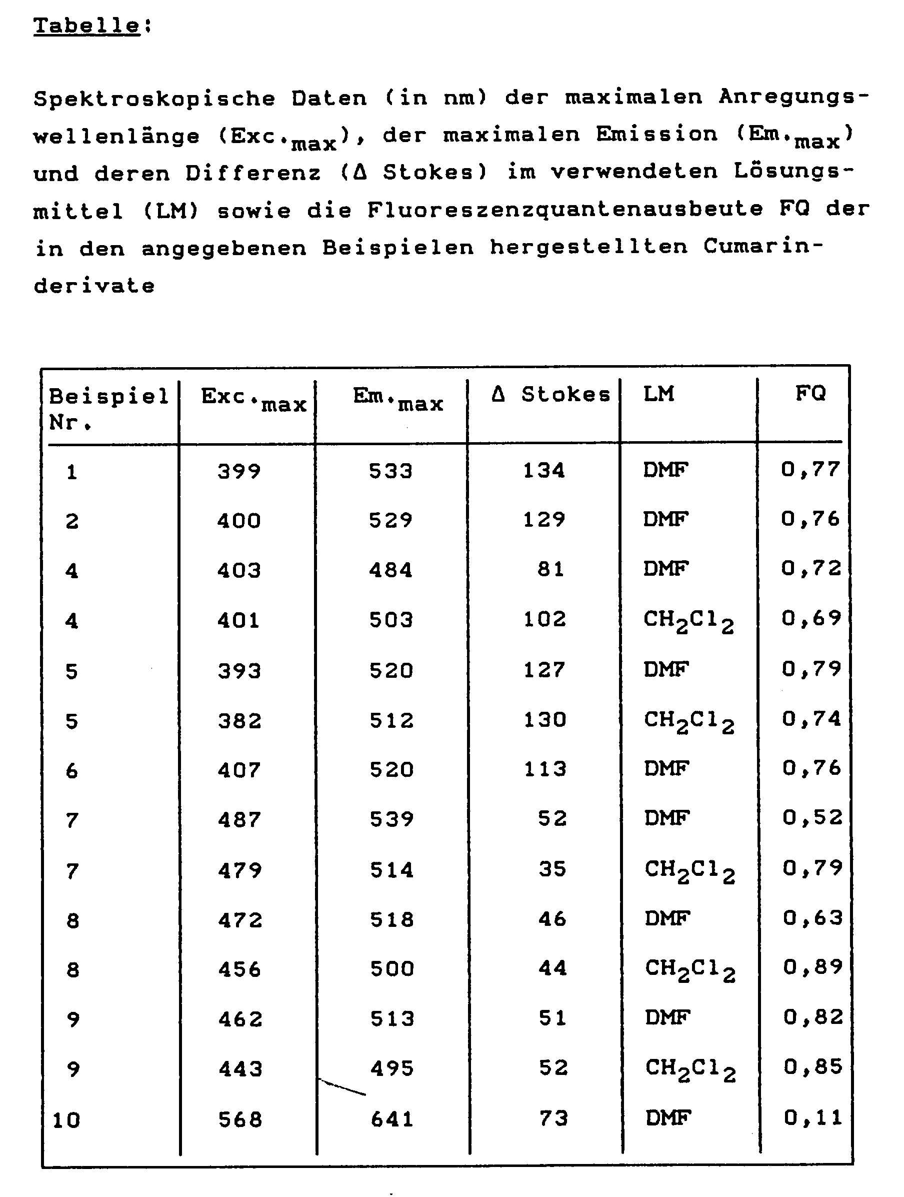

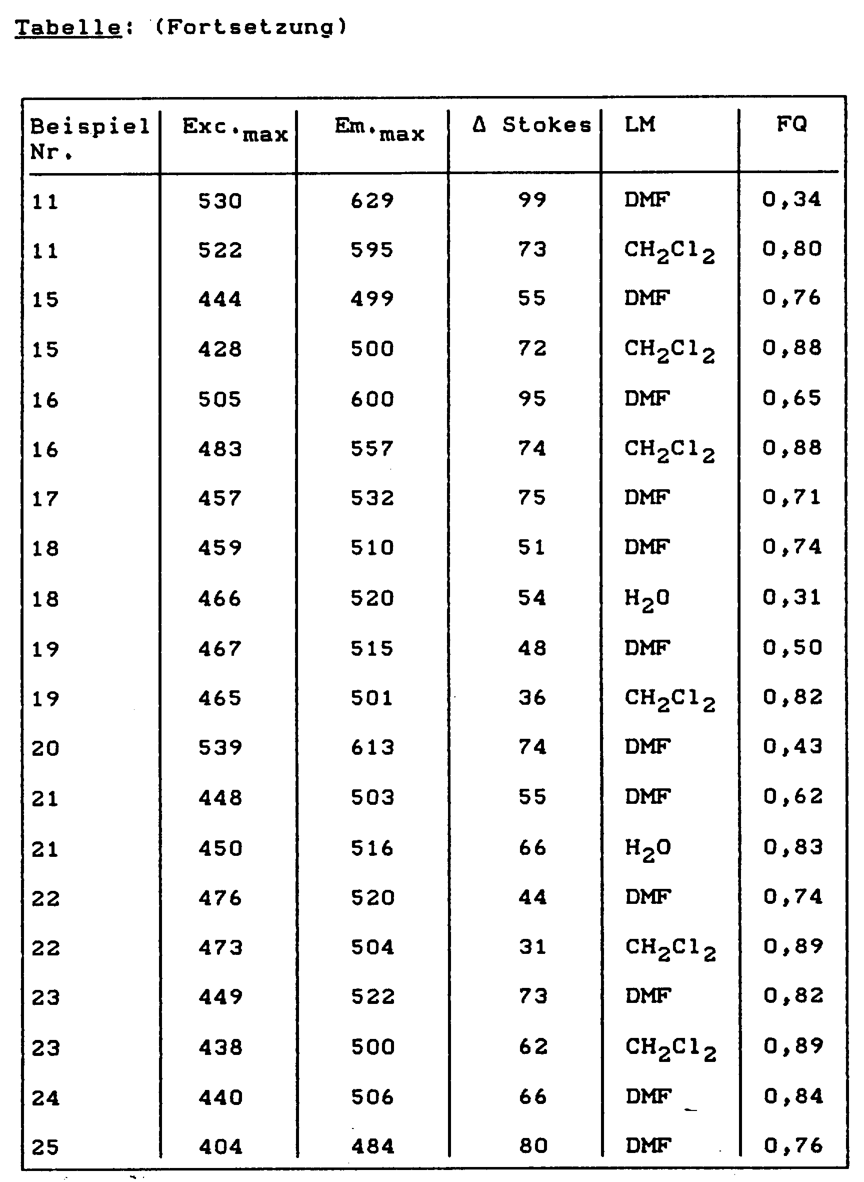

## Patentansprüche

1. Cumarinderivate der Formel 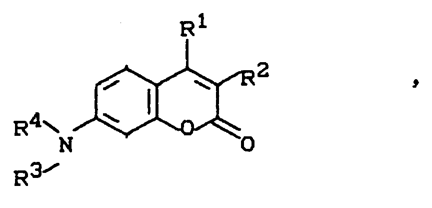 in der
R¹ Wasserstoff oder Cyano bedeutet,
R für Phenyl oder für in 2-, 4- oder 5-Stellung gebundenes Thiazolyl steht, wobei Phenyl durch Cyano, Amino, -NH-C₁-C₄-Alkyl, -C₁-C₄-Alkyl-NH₂, -C₁-C₄-Alkyl-NH-C₁-C₄-Alkyl, Carboxyl, C₁-C₄-Alkoxy-carbonyl, C₁-C₄-Alkyl-carbonyl-oxy, Hydroxy, C₁-C₄-Alkylamino-carbonyl oder C₁-C₄-Alkylcarbonyl-amino substituiert ist und zusätzlich durch C₁-C₄-Alkyl, Fluor, Chlor oder Brom substituiert sein kann, und wobei Thiazolyl ein- oder zweifach durch Chlor, Cyano, Carboxyl oder C₁-C₄-Alkoxy-carbonyl substituiert ist, wobei bei zweifacher Substitution die beiden Substituenten verschieden sein können und wobei die 4- und die 5-Stellung gemeinsam einen ankondensierten Benzolring tragen können, der durch Carboxyl, Amino oder Hydroxy substituiert sein kann,
R³ Wasserstoff, C₁-C₄-Alkyl oder C₁-C₄-Alkoxy-carbonyl-C₁-C₄-alkyl bedeutet und
R⁴ für C₁-C₄-Alkyl oder Phenylsulfonyl steht, wobei C₁-C₄-Alkyl durch Hydroxy, Amino, Carboxy oder C₁-C₄-Alkoxy-carbonyl substituiert sein kann und Phenyl ein- oder zweifach durch Chlor, Brom oder C₁-C₄-Alkyl substituiert sein kann,
wobei weiterhin R³ und R⁴ gemeinsam mit dem N-Atom, das sie substituieren, einen Morpholinring oder einen Piperazinring, der einen oder zwei Substituenten aus der Gruppe Methyl, Ethyl und Phenyl tragen kann, bedeuten können und
wobei weiterhin einer der Reste R, R³ und R⁴ eine primäre oder sekundäre Aminogruppe, die Hydroxylgruppe, die Carboxylgruppe, Cyano, Acylamino Phenylsulfonylamino oder die C₁-C₄-Alkoxycarbonylgruppe trägt, wobei Cyano oder die C₁-C₄-Alkoxycarbonylgruppe durch Verseifung in die Carboxylgruppe, ebenfalls durch Verseifung die Acylamino- oder Phenylsulfonylaminogruppe in eine Aminogruppe und die Cyanogruppe durch Hydrierung in eine Primäre Aminogruppe überführt werden kann.

2. Verfahren zur Herstellung von Cumarinderivaten der Formel 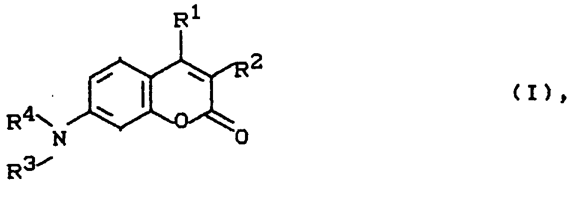 in der
R¹ Wasserstoff oder Cyano bedeutet,
R für Phenyl oder für in 2-, 4- oder 5-Stellung gebundenes Thiazolyl steht, wobei Phenyl durch Cyano, Amino, -NH-C₁-C₄-Alkyl, -C₁-C₄-Alkyl-NH₂, -C₁-C₄-Alkyl-NH-C₁-C₄-Alkyl, Carboxyl, C₁-C₄-Alkoxy-carbonyl, C₁-C₄-Alkyl-carbonyloxy, Hydroxy, C₁-C₄-Alkylamino-carbonyl oder C₁-C₄-Alkylcarbonyl-amino substituiert ist und zusätzlich durch C₁-C₄-Alkyl, Fluor, Chlor oder Brom substituiert sein kann, und wobei Thiazolyl ein- oder zweifach durch Chlor, Cyano, Carboxyl oder C₁-C₄-Alkoxy-carbonyl substituiert ist, wobei bei zweifacher Substitution die beiden Substituenten verschieden sein können und wobei die 4- und die 5-Stellung gemeinsam einen ankondensierten Benzolring tragen können, der durch Carboxyl, Amino oder Hydroxy substituiert sein kann,
R³ Wasserstoff, C₁-C₄-Alkyl oder C₁-C₄-Alkoxy-carbonyl-C₁-C₄-alkyl bedeutet und
R⁴ für C₁-C₄-Alkyl oder Phenylsulfonyl steht, wobei C₁-C₄-Alkyl durch Hydroxy, Amino, Carboxy oder C₁-C₄-Alkoxy-carbonyl substituiert sein kann und Phenyl ein- oder zweifach durch Chlor, Brom oder C₁-C₄-Alkyl substituiert sein kann,
wobei weiterhin R³ und R⁴ gemeinsam mit dem N-Atom, das sie substituieren, einen Morpholinring oder einen Piperazinring, der einen oder zwei Substituenten aus der Gruppe Methyl, Ethyl und Phenyl tragen kann, bedeuten können und
wobei weiterhin einer der Reste R, R³ und R⁴ eine primäre oder sekundäre Aminogruppe, die Hydroxylgruppe, die Carboxylgruppe oder die C₁-C₄-Alkoxy-carbonylgruppe trägt oder durch Verseifung von Cyano oder C₁-C₄-Alkoxy-carbonyl in die Carboxylgruppe, durch Verseifung von Acylamino oder Phenylsulfonylamino in eine Aminogruppe oder durch Hydrierung von Cyano in eine primäre Aminogruppe übergeführt werden kann,
dadurch gekennzeichnet, daß man
a) ein m-Aminophenol und ein Formylessigsäurederivat der Formeln 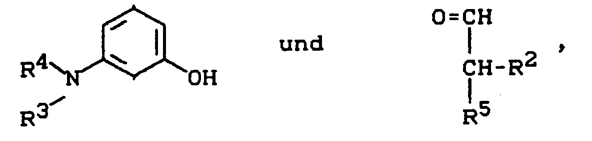
worin
R, R³ und R⁴ den obigen Bedeutungsumfang haben
und
R⁵ Cyano, C₁-C₄-Alkoxy-carbonyl oder Carboxyl bedeutet,
miteinander umsetzt oder
b) einen Salicylaldehyd und ein Essigsäurederivat der Formeln 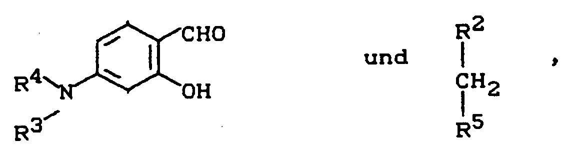
worin
R, R³, R⁴ und R⁵ den obigen Bedeutungsumfang haben
miteinander umsetzt,
wobei im Falle von R⁵ = CN bei a) und b) zunächst ein Imino-Zwischenprodukt der Formel 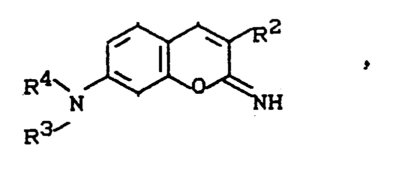
worin R, R³ und R⁴ den obigen Bedeutungsumfang haben,
entsteht und dieses Imino-Zwischenprodukt unter Abspaltung der Iminogruppen hydrolysiert wird, oder
c) für den Fall, daß R¹ Cyano bedeutet, das Imino-Zwischenprodukt gemäß b) oder das Cumarinderivat der Formel (I) mit Cyanidionen zum Imino-Cyano-Zwischenprodukt oder zum Cyano-Zwischenprodukt der Formeln 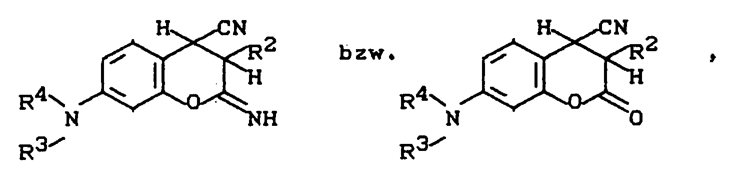 worin R, R³ und R⁴ die obige Bedeutung haben,
umsetzt und dieses Zwischenprodukt zum Cumarinderivat oxidiert und gegebenenfalls zusätzlich hydrolysiert.

3. 2-Thiazolyl-essigsäurederivate der Formel 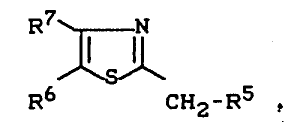
in der
R⁶ Chlor oder Cyano und
R⁷ Chlor bedeuten und
R⁵ für Carboxyl, C₁-C₄-Alkoxy-carbonyl oder Cyano steht.

4. Cumarinderivate der Formel 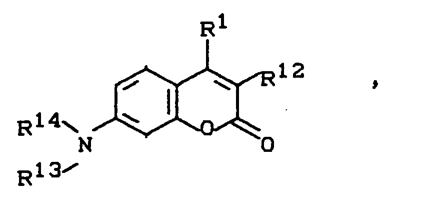
in der
R¹ Wasserstoff oder Cyano bedeutet,
R¹ für Phenyl oder für in 2-, 4- oder 5-Stellung gebundenes Thiazolyl steht, wobei Phenyl durch Carboxyl, C₁-C₄-Alkyl-carbonyloxy, Amino, -NH-C₁-C₄-Alkyl, -C₁-C₄-Alkyl-NH₂, C₁-C₄-Alkyl, Cyano, Fluor, Chlor oder Brom substituiert sein kann und wobei Thiazolyl durch Chlor, Cyano oder Carboxyl oder einen in 4 und 5-Stellung ankondensierten Benzolring, der seinerseits Carboxyl oder Amino tragen kann, substituiert sein kann,
R¹³ Wasserstoff, Methyl oder Ethyl bedeutet und
R¹⁴ für -C₁-C₄-Alkyl-OH, -C₁-C₄-Alkyl-NH₂ oder C₁-C₄-Alkyl-COOH steht,
wobei weiterhin R¹³ und R¹⁴ gemeinsam mit dem N-Atom, das sie substituieren, einen Morpholinring oder einen Piperazinring, der durch Methyl, Phenyl oder Methyl und Phenyl substituiert sein kann, bedeuten können.

5. Cumarinderivate der Formel 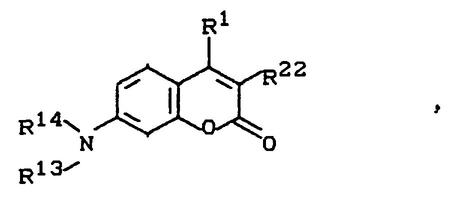 in der
R¹, R¹³ und R¹⁴ den in Anspruch 4 genannten Bedeutungsumfang annehmen und
R für Phenyl oder für in 2-Stellung gebundenes Thiazolyl steht, wobei Phenyl durch para-Carboxyl, para-Amino, para-NH-C₁-C₄-Alkyl, para-CH₂-NH₂, Cyano, Methyl oder Ethyl substituiert sein kann und wobei Thiazolyl durch Chlor, Cyano oder Carboxyl oder einen in 4- und 5-Stellung ankondensierten Benzolring, der seinerseits Carboxyl oder Amino tragen kann, substituiert sein kann.

6. Verwendung der Cumarinderivate gemäß Anspruch 1 zum Anfärben von biologisch aktiven Verbindungen.

## Claims

1. Coumarin derivatives of the formula 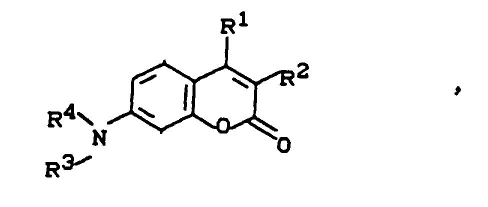 in which
R¹ denotes hydrogen or cyano,
R represents phenyl or thiazolyl bonded in the 2-, 4- or 5-position, where phenyl is substituted by cyano, amino, -NH-C₁-C₄-alkyl, -C₁-C₄-alkyl-NH₂, -C₁-C₄-alkyl-NH-C₁-C₄-alkyl, carboxyl, C₁-C₄-alkoxy-carbonyl, C₁-C₄-alkyl-carbonyloxy, hydroxyl, C₁-C₄-alkylamino-carbonyl or C₁-C₄-alkylcarbonyl-amino and can additionally be substituted by C₁-C₄-alkyl, fluorine, chlorine or bromine, and where thiazolyl is monosubstituted or disubstituted by chlorine, cyano, carboxyl or C₁-C₄-alkoxy-carbonyl, where, in the case of disubstitution, the two substituents may be different and where the 4- and the 5-position can together carry a fused benzene ring which can be substituted by carboxyl, amino or hydroxyl,
R³ denotes hydrogen, C₁-C₄-alkyl or C₁-C₄-alkoxy-carbonyl-C₁-C₄-alkyl and
R⁴ represents C₁-C₄-alkyl or phenylsulphonyl, where C₁-C₄-alkyl can be substituted by hydroxyl, amino, carboxyl or C₁-C₄-alkoxy-carbonyl and phenyl can be monosubstituted or disubstituted by chlorine, bromine or C₁-C₄-alkyl,
where furthermore R³ and R⁴, together with the N atom which they substitute, can denote a morpholine ring or a piperazine ring which can carry one or two substituents from the group consisting of methyl, ethyl and phenyl, and
where furthermore one of the radicals R, R³ and R⁴ carries a primary or secondary amino group, the hydroxyl group, the carboxyl group, cyano, acylamino phenylsulphonylamino or the C₁-C₄-alkoxy-carbonyl group, it being possible to convert cyano or the C₁-C₄-alkoxycarbonyl group into the carboxyl group by hydrolysis, likewise the acylamino group or the phenylsulphonylamino group into an amino group by hydrolysis and the cyano group into a primary amino group by hydrogenation.

2. Process for the preparation of coumarin derivatives of the formula 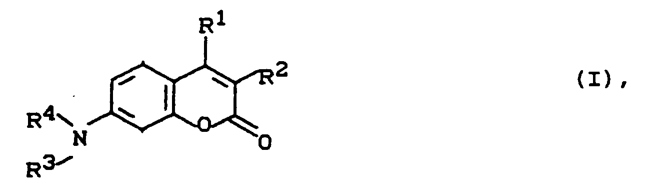 in which
R¹ denotes hydrogen or cyano,
R represents phenyl or thiazolyl bonded in the 2-, 4- or 5-position, where phenyl is substituted by cyano, amino, -NH-C₁-C₄-alkyl, -C₁-C₄-alkyl-NH₂, -C₁-C₄-alkyl-NH-C₁-C₄-alkyl, carboxyl, C₁-C₄-alkoxy-carbonyl, C₁-C₄-alkyl-carbonyloxy, hydroxyl, C₁-C₄-alkylamino-carbonyl or C₁-C₄-alkylcarbonyl-amino and can additionally be substituted by C₁-C₄-alkyl, fluorine, chlorine or bromine, and where thiazolyl is monosubstituted or disubstituted by chlorine, cyano, carboxyl or C₁-C₄-alkoxy-carbonyl, where, in the case of disubstitution, the two substituents may be different and where the 4- and the 5-position can together carry a fused benzene ring which can be substituted by carboxyl, amino or hydroxyl,
R³ denotes hydrogen, C₁-C₄-alkyl or C₁-C₄-alkoxy-carbonyl-C₁-C₄-alkyl and
R⁴ represents C₁-C₄-alkyl or phenylsulphonyl, where C₁-C₄-alkyl can be substituted by hydroxyl, amino, carboxyl or C₁-C₄-alkoxy-carbonyl and phenyl can be monosubstituted or disubstituted by chlorine, bromine or C₁-C₄-alkyl,
where furthermore R³ and R⁴, together with the N atom which they substitute, can denote a morpholine ring or a piperazine ring which can carry one or two substituents from the group consisting of methyl, ethyl and phenyl, and
where furthermore one of the radicals R, R³ and R⁴ carries a primary or secondary amino group, the hydroxyl group, the carboxyl group or the C₁-C₄-alkoxy-carbonyl group or can be converted into the carboxyl group by hydrolysis of cyano or C₁-C₄-alkoxy-carbonyl, into an amino group by hydrolysis of acylamino or phenylsulphonylamino or into a primary group by hydrogenation of cyano,
characterized in that
a) an m-aminophenol and a formylacetic acid derivative of the formulae 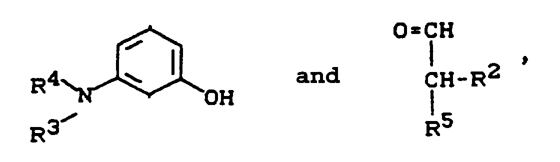
in which
R, R³ and R⁴ have the above scope of meaning
and
R⁵ denotes cyano, C₁-C₄-alkoxy-carbonyl or carboxyl,
are reacted with one another or
b) a salicylaldehyde and an acetic acid derivative of the formulae 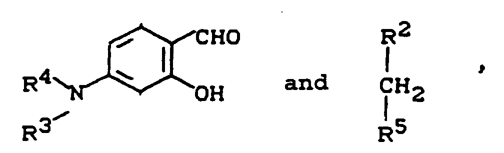
in which
R, R³, R⁴ and R⁵ have the above scope of meaning,
are reacted with one another,
where if R⁵ = CN in a) and b), first an imino intermediate of the formula 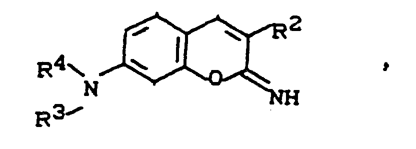
in which R, R³ and R⁴ have the above scope of meaning,
is formed and this imino intermediate is hydrolysed with elimination of the imino group, or
c) in the case in which R¹ denotes cyano, the imino intermediate as in b) or the coumarin derivative of the formula (I) is reacted with cyanide ions to give the imino-cyano intermediate or the cyano intermediate of the formulae 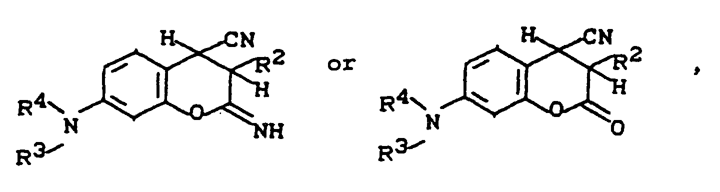 in which R, R³ and R⁴ have the above scope of meaning,
and this intermediate is oxidized to the coumarin derivative and optionally additionally hydrolysed.

3. 2-Thiazolyl-acetic acid derivatives of the formula 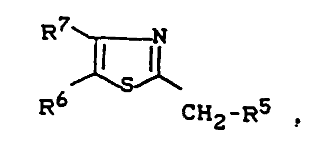 in which
R⁶ denotes chlorine or cyano and
R⁷ denotes chlorine and
R⁵ represents carboxyl, C₁-C₄-alkoxy-carbonyl or cyano.

4. Coumarin derivatives of the formula 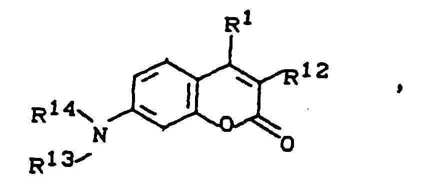 in which
R¹ denotes hydrogen or cyano,
R¹ represents phenyl or thiazolyl bonded in the 2-, 4- or 5-position, where phenyl can be substituted by carboxyl, C₁-C₄-alkyl-carbonyloxy, amino, -NH-C₁-C₄-alkyl, -C₁-C₄-alkyl-NH₂, C₁-C₄-alkyl, cyano, fluorine, chlorine or bromine and where thiazolyl can be substituted by chlorine, cyano or carboxyl or a benzene ring fused in the 4- and 5-position, which in turn can carry carboxyl or amino,
R¹³ denotes hydrogen, methyl or ethyl and
R¹⁴ represents -C₁-C₄-alkyl-OH, -C₁-C₄-alkyl-NH₂ or C₁-C₄-alkyl-COOH,
where furthermore R¹³ and R¹⁴, together with the N atom which they substitute, can denote a morpholine ring or a piperazine ring which can be substituted by methyl, phenyl or methyl and phenyl.

5. Coumarin derivatives of the formula 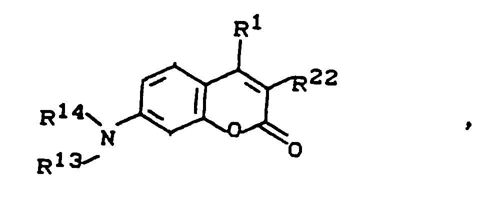 in which
R¹, R¹³ and R¹⁴ assume the scope of meaning mentioned in Claim 4 and
R represents phenyl or thiazolyl bonded in the 2-position, where phenyl can be substituted by para-carboxyl, para-amino, para-NH-C₁-C₄-alkyl, para-CH₂-NH₂, cyano, methyl or ethyl and where thiazolyl can be substituted by chlorine, cyano or carboxyl or a benzene ring fused in the 4-and 5-position which in turn can carry carboxyl or amino.

6. Use of the coumarin derivatives according to Claim 1 for dyeing biologically active compounds.

## Revendications

1. Dérivés de la coumarine répondant à la formule : 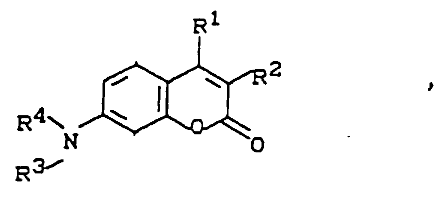 dans laquelle
R¹ représente l'hydrogène ou un groupe cyano,
R représente un groupe phényle ou un groupe thiazolyle relié en position 2, 4 ou 5,
le groupe phényle étant substitué par des groupes cyano, amino, -NH-alkyle en C₁-C₄, (alkyle en C₁-C₄)-NH₂, (alkyle en C₁-C₄)-NH-alkyle en C₁-C₄, carboxyle, (alcoxy en C₁-C₄)-carbonyle, (alkyle en C₁-C₄)-carbonyloxy, hydroxy, (alkylamino en C₁-C₄)-carbonyle ou (alkyle en C₁-C₄)-carbonylamino et pouvant en outre être substitué par des groupes alkyle en C₁-C₄, le fluor, le chlore ou le brome, le groupe thiazolyle portant un ou deux substituants chloro, cyano, carboxyle ou (alcoxy en C₁-C₄) carbonyle, les deux substituants pouvant être différents dans le cas d'une disubstitution, les positions 4 et 5 pouvant en outre porter ensemble un cycle benzénique condensé qui peut lui-même être substitué par des groupes carboxyle, amino ou hydroxy,
R³ représente l'hydrogène, un groupe alkyle en C₁-C₄ ou (alcoxy en C₁-C₄)-carbonylalkyle en C₁-C₄ et
R⁴ représente un groupe alkyle en C₁-C₄ ou phénylsulfonyle, le groupe alkyle en C₁-C₄ pouvant être substitué par des groupes hydroxy, arnino, carboxy ou (alcoxy en C₁-C₄)-carbonyle, et le groupe phényle pouvant porter un ou deux substituants chloro, bromo ou alkyle en C₁-C₄,
R³ et R⁴ pouvant également former ensemble et avec l'atome d'azote dont ils sont substituants un cycle morpholine ou un cycle pipérazine qui peut porter un ou deux substituants choisis parmi les groupes méthyle, éthyle et phényle, et
d'autre part, l'un des substituants R, R³ et R⁴ porte un groupe amino primaire ou secondaire, un groupe hydroxy, un groupe carboxyle, cyano, acylamino, phénylsulfonylamino ou (alcoxy en C₁-C₄)-carbonyle, le groupe cyano ou le groupe (alcoxy en C₁-C₄)-carbonyle pouvant être converti par saponification en un groupe carboxyle, et de même le groupe acylamino ou le groupe phénylsulfonylamino pouvant être converti par saponification en un groupe amino et le groupe cyano par hydrogénation en un groupe amino primaire.

2. Procédé de préparation des dérivés de la coumarine répondant à la formule : 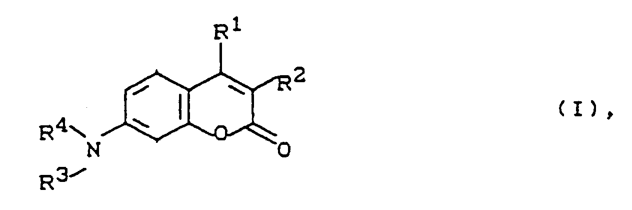 dans laquelle
R¹ représente l'hydrogène ou un groupe cyano,
R représente un groupe phényle ou un groupe thiazolyle relié en position 2, 4 ou 5,
le groupe phényle étant substitué par des groupes cyano, amino, -NH-alkyle en C₁-C₄, (alkyle en C₁-C₄)-NH₂, (alkyle en C₁-C₄)-NH-alkyle en C₁-C₄, carboxyle, (alcoxy en C₁-C₄)-carbonyle, (alkyle en C₁-C₄)-carbonyloxy, hydroxy, (alkylamino en C₁-C₄)-carbonyle ou (alkyle en C₁-C₄)-carbonylamino et pouvant en outre être substitué par des groupes alkyle en C₁-C₄, le fluor, le chlore ou le brome, le groupe thiazolyle portant un ou deux substituants chloro, cyano, carboxyle ou (alcoxy en C₁-C₄) carbonyle, les deux substituants pouvant être différents dans le cas d'une disubstitution, les positions 4 et 5 pouvant en outre porter ensemble un cycle benzénique condensé qui peut lui-même être substitué par des groupes carboxyle, amino ou hydroxy,
R³ représente l'hydrogène, un groupe alkyle en C₁-C₄ ou (alcoxy en C₁-C₄)-carbonylalkyle en C₁-C₄ et
R⁴ représente un groupe alkyle en C₁-C₄ ou phénylsulfonyle, le groupe alkyle en C₁-C₄ pouvant être substitué par des groupes hydroxy, amino, carboxy ou (alcoxy en C₁-C₄)-carbonyle, et le groupe phényle pouvant porter un ou deux substituants chloro, bromo ou alkyle en C₁-C₄,
R³ et R⁴ pouvant également former ensemble et avec l'atome d'azote dont ils sont substituants un cycle morpholine ou un cycle pipérazine qui peut porter un ou deux substituants choisis parmi les groupes méthyle, éthyle et phényle, et
d'autre part, l'un des substituants R, R³ et R⁴ porte un groupe amino primaire ou secondaire, un groupe hydroxy, un groupe carboxyle, cyano, acylamino, phénylsulfonylamino ou (alcoxy en C₁-C₄)-carbonyle, le groupe cyano ou le groupe (alcoxy en C₁-C₄)-carbonyle pouvant être converti par saponification en un groupe carboxyle, et de même le groupe acylamino ou le groupe phénylsulfonylamino pouvant être converti par saponification en un groupe amino et le groupe cyano par hydrogénation en un groupe amino primaire,
caractérisé en ce que
a) on fait réagir entre eux un m-aminophénol et un dérivé de l'acide formylacétique de formules : 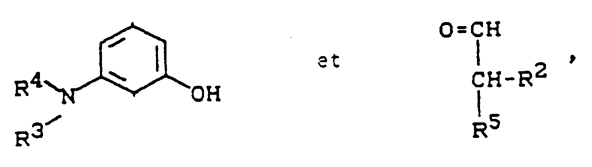 dans lesquelles
R, R³ et R⁴ ont les significations indiquées ci-dessus et
R⁵ représente un groupe cyano, (alcoxy en C₁-C₄)-carbonyle ou carboxyle,
ou bien
b) on fait réagir entre eux un aldéhyde salicylique et un dérivé de l'acide acétique de formules 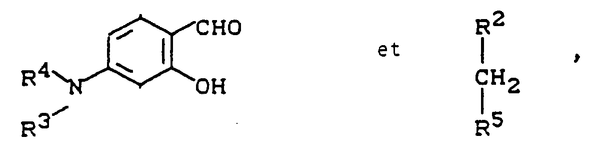 dans lesquelles
R, R³, R⁴ et R⁵ ont les significations indiquées ci-dessus,
étant précisé que, dans le cas où R⁵ = CN, aussi bien en a) qu'en b), on forme d'abord un produit intermédiaire iminé de formule : 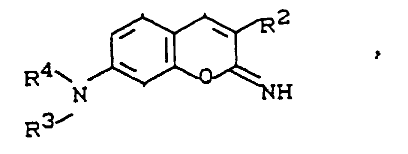 dans laquelle
R, R³ et R⁴ ont les significations indiquées ci-dessus,
qu'on hydrolyse avec scission des groupes imino, ou bien
c) dans le cas où R¹ représente un groupe cyano, on fait réagir le produit intermédiaire iminé selon b) ou le dérivé de la coumarine de formule (I) avec des ions cyanure, ce qui donne un produit intermédiaire iminocyané ou respectivement un produit intermédiaire cyané de formules : 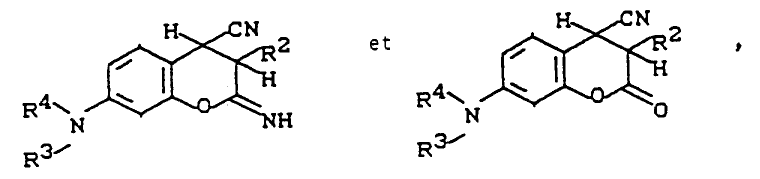 dans lesquelles
R, R³ et R⁴ ont les significations indiquées ci-dessus,
qu'on oxyde en le dérivé de la coumarine en faisant suivre le cas échéant d'une hydrolyse.

3. Dérivés de l'acide 2-thiazolylacétique répondant à la formule : 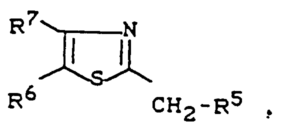 dans laquelle
R⁶ représente le chlore ou un groupe cyano,
R⁷ représente le chlore et
R⁵ représente un groupe carboxyle, (alcoxy en C₁-C₄)-carbonyle ou cyano.

4. Dérivés de la coumarine répondant à la formule : 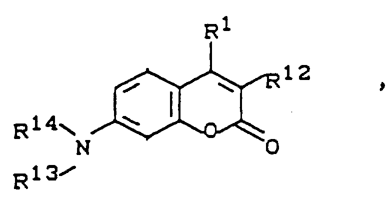 dans laquelle
R¹ représente l'hydrogène ou un groupe cyano,
R¹ représente un groupe phényle ou thiazolyle relié en position 2, 4 ou 5, le groupe phényle pouvant être substitué par des groupes carboxyle, (alkyle en C₁-C₄)-carbonyloxy, amino, -NH-alkyle en C₁-C₄, -(alkyle en C₁-C₄)-NH₂, alkyle en C₁-C₄, cyano, le fluor, le chlore ou le brome, et le groupe thiazolyle pouvant être substitué par le chlore, un groupe cyano ou carboxyle ou par un cycle benzénique condensé en position 5 et qui peut lui-même porter des groupes carboxyle ou amino,
R¹³ représente l'hydrogène, un groupe méthyle ou éthyle et
R¹⁴ représente un groupe -(alkyle en C₁-C₄)-OH, -(alkyle en C₁-C₄)-NH₂ ou (alkyle en C₁-C₄)-COOH,
R¹³ et R¹⁴ pouvant en outre former ensemble et avec l'atome d'azote dont ils sont substituants un cycle morpholine ou pipérazine qui peut être substitué par des groupes méthyle, des groupes phényle, ou des groupes méthyle et phényle.

5. Dérivés de la coumarine de formule : 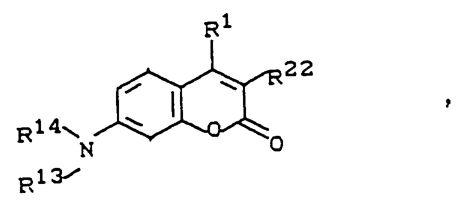 dans laquelle
R¹, R¹³ et R¹⁴ ont les significations indiquées dans la revendication 4 et
R représente un groupe phényle ou un groupe thiazolyle relié en position 2, le groupe phényle pouvant porter des substituants para-carboxyle, para-amino, para-NH-alkyle en C₁-C₄, para-CH₂-NH₂, cyano, méthyle ou éthyle, et le groupe thiazolyle pouvant être substitué par le chlore, un groupe cyano ou carboxyle ou un cycle benzénique condensé sur les positions 4 et 5 et qui peut lui-même porter des substituants carboxyles ou amino.

6. Utilisation des dérivés de la coumarine selon revendication 1 pour la coloration de composés possédant une activité biologique.
